# EUROPEAN PATENT SPECIFICATION

(11) **EP 2 440 071 B1**
(45) Date of publication and mention of the grant of the patent: **29.03.2017**
(21) Application number: 10728334.3
(22) Date of filing: 08.06.2010
(51) Int. Cl.: A23G 4/10, A23G 4/06, A23L 29/30, A23L 27/30

(54) **Extruded particles**
Extrudierte Partikel
Particules extrudées

(30) Priority: 08.06.2009 EP 09162137
(43) Date of publication of application: 18.04.2012
(73) Proprietor: Firmenich S.A., 1211 Geneva 8 (CH)
(72) Inventor: BARRA, Jérôme, F-74160 Neydens (FR); BOUQUERAND, Pierre-Etienne, F-74930 Pers-jussy (FR); ZAMPIERI, Dana, CH-1257 Landecy (CH)
(74) Representative: Holmes, Michael Anthony
(86) International application number: PCT/IB2010/052528
(87) International publication number: WO 2010/143126

(56) References cited:
- EP-A- 0 272 220
- WO-A-91/03147
- WO-A-2008/002691
- US-A- 4 933 190
- US-A1- 2002 164 397
- US-A1- 2007 116 800

## Description

### Technical Field

The present invention relates to extruded particles comprising an encapsulated sweetening component. It also relates to a foodstuff comprising such extruded particles.

### Background and Prior Art

Much effort has been directed to prolonging flavour release and perception in food products and particularly in chewing gum compositions. Activity in this area has focused on modifying the flavour composition with increased emphasis on longer lasting base or middle notes, increasing the quantity of flavour present or providing the flavour in an encapsulated form.

It is also known to encapsulate sweeteners in chewing gum applications to provide longer lasting sweetness.

For instance, US 4597970 discloses a chewing gum composition comprising a gum base; an agglomerated sweetener delivery system capable of effecting a controlled release of core material comprising at least one natural or artificial core material selected from the group consisting of amino acid based sweeteners, dipeptide sweeteners, glycyrrhizin, saccharin and its salts, acesulfame salts, cyclamates, steviosides, talin, dihydrochalcone compounds, flavouring agents and mixtures thereof; a hydrophobic matrix consisting essentially of lecithin and an edible material having a melting point in the range of about 25° C. to about 100° C. selected from the group consisting of (a) fatty acids having an iodine value of about 1 to about 10, (b) natural waxes, (c) synthetic waxes and (d) mixtures thereof; and at least one glyceride.

WO-A-84/00320 discloses a food-grade shellac encapsulant for active chewing gum ingredients. Encapsulation of sweetener, flavouring agent, food grade acid and pharmaceutical agents to achieve a gradual and controlled release of such ingredients is described. The ratio of shellac to sweetener such as aspartame is about 1:20 to about 0.9:1.

US 4911934 discloses a chewing gum composition containing a sweetening agent encapsulated in a coating material comprising a hydrophobic polymer, such as a cellulose ether, and a hydrophobic plasticizer. Encapsulation is preferably performed by forming a wet mix of the ingredients and then oven drying to form granules. The encapsulated sweetening agent is said to give sustained release of sweetener.

US-A-2004/0180068 refers to cellulose-based particles and liquids as well as methods for their preparation. The particles comprise flavour as a plasticizer and a non-polar active ingredient.

US-A-2005/0220867 discloses a delivery system for active component as part of an edible composition having a preselected tensile strength. Edible compositions include chewing gum and the active component may be a high intensity sweetener.

None of these documents refer to the use of an encapsulated sweetening agent to enhance the longevity and intensity of flavour perception.

In addressing the abovementioned issues, it is further desirable to avoid encapsulation systems which require additional solvents. Thus, for instance, spray-coating should be avoided.

Furthermore, sweetening ingredients that are either too hydrophobic or too hydrophilic are less desirable. In particular, when the sweetener is highly hydrophilic, it will be released too quickly in a chewing gum and so will not provide the desired flavour release profile. This is a known problem for sweetening agents such as acesulfame-K. For instance, document DE-A1-3120857 discloses the use of acesulfame-K in chewing gum and describes the sensory properties as rapid, perceptible and clear sweetness. WO-A1-96/20608 also refers to the release characteristics of acesulfame-K and discloses a method to control the release of acesulfame-K in chewing gum by coating and drying. However, this entraps the sweetener in the gum base, and so the sweetener remains entrapped as long as the integrity of the carrier is maintained.

Similarly, when the sweetener is very hydrophobic, it will be released very slowly in a chewing gum. This is known characteristic of neohesperidin dihydrochalcone. The time/intensity profile of neohesperidin dihydrochalcone is characterized by a delayed onset and long duration of sweetness perception (Marie-Odile Portmann and David Kilcast, Food Chemistry Volume 56, Issue 3, July 1996, Pages 291-302; G.A Crosby et al in: Developments in Sweeteners, C.A.M Hough et al (Ed), 1, 135-164. Applied Science Publishers Ltd, London, 1979; G.E. Dubois et al: Journal of Agricultural and Food Chemistry 1981 29 1269-1276).

Furthermore, when such a sweetener is entrapped into a hydrophobic carrier, it will remain in the gum base as long as the integrity of the carrier is maintained, and, upon chewing, the sweetener at the surface of the hydrophobic carrier interacts with the gum, thus delaying release in the mouth. The sweetener within the swelling front of the gum base will be released even more slowly as it will interact with the matrix system. Consequently, when such a sweetener is used, there is no beneficial effect when using an encapsulated system.

EP 0 272 220 (Schobel *et at*) relates to the encapsulation of a sweetening agent in a coating material including a hydrophobic polymer and a hydrophobic plasticizer and chewing gum composition containing said sweetening agent.

WO 2008/002691 (Barrera et al) relates to a method of manufacturing a delivery system of at least one active component encapsulated in an encapsulating material, which is useful, for example, for providing delayed and/or controlled release of the active.

The present invention seeks to address one or more of the abovementioned problems and/or to provide one or more of the abovementioned benefits.

### Summary of the Invention

Accordingly, the present invention provides an extruded particle for use in a foodstuff, the extruded particle comprising a carrier matrix, the matrix comprising ethyl cellulose and a hydrophobic plasticizer which is oleic acid, and a sweetening component selected from the group consisting of stevioside, rebaudioside A, sodium cyclamate, aspartame, sucralose and sodium saccharine, or mixtures thereof dispersed throughout the matrix.

In another aspect, the invention provides a foodstuff, comprising the abovementioned extruded particle.

In a further aspect, the invention provides the use of the extruded particle to prolong or sustain the perception of a flavour during chewing or consumption of the foodstuff.

In yet another aspect the invention provides a method of preparing extruded particles comprising the steps of extruding a mixture comprising ethyl cellulose, a hydrophobic plasticizer which is oleic acid, and the sweetening component selected from the group consisting of stevioside, rebaudioside A, sodium cyclamate, aspartame, sucralose and sodium saccharine, or mixtures thereof, and granulating the extruded material to form particles.

### Detailed Description of the Invention

The present invention relates to extruded particles for use in a foodstuff, the extruded particles comprising a carrier matrix for the sweetening agent.

### Matrix Component

The matrix component or carrier for the sweetening component comprises ethyl cellulose and a hydrophobic plasticizer which is oleic acid. More preferably, the carrier consists essentially of ethyl cellulose and a hydrophobic plasticizer which is oleic acid.

It is especially important that the matrix comprises these components since this provides the desired release profile of the sweetening component and thus enables the longer-lasting flavor or sweetness perception that is desired. Such a release profile is not typically seen with conventional matrices used in extrusion, such as those based primarily on maltodextrin having a DE of between 5 and 20.

Ethyl cellulose is a derivative of cellulose in which a certain number of the hydroxyl groups on the repeating glucose backbone are converted into ethyl groups. The number of groups converted is also referred to as the degree of substitution.

In the context of the present invention, the degree of substitution per glucose repeat unit is preferably from 2 to 3, more preferably from 2.22 to 2.81. At this degree of substitution, it is found that the ethyl cellulose provides a very stable matrix.

It is preferred that the viscosity of the ethyl cellulose is from 50 mPa.s to 1'000 mPa.s, more preferably 75 mPa.s to 750 mPa.s, most preferably 100 mPa.s to 500 mPa.s, measured as a 5% solution based on 80% toluene 20% ethanol, at 25°C in an Ubbelohde viscometer.

The molecular weight of the cellulose ether derivative is preferably within the range of from 50'000 to 2'000'000, more preferably from 75'000 to 1'500'000, most preferably from 100'000 to 1'250'000.

Commercially available ethyl cellulose suitable for use in the present invention includes, for instance, the Klucel® range, ex Aqualon-Hercules.

The ethyl cellulose may be used in combination with compatible carriers. However, it should preferably not be used in combination with other cellulose derivatives. For instance, hydroxypropyl cellulose cannot readily be plasticized with hydrophobic plasticizers, such as oils or terpenes, and is unacceptable for use in the extruded product of the present invention.

The amount of ethyl cellulose is preferably from 95 to 40% by weight, based on the total weight of the extruded particle, more preferably 80 to 40%, most preferably 75 to 50%.

### Hydrophobic Plasticizer

A further essential component of the matrix is an effective amount of a plasticizer for the ethyl cellulose. The plasticizer facilitates the extrusion process which, in its absence, would be exceptionally difficult or even impossible.

The plasticizer, which is oleic acid, is hydrophobic. Hydrophilic plasticizers are unacceptable for use in the present invention as they do not facilitate the extrusion process with the ethyl cellulose matrix and do not afford an extruded particle having the desired soft texture.

The oleic acid plasticizer is liquid at 25°C since this also enables the formation of particles which are compatible with foodstuffs where the desired texture is soft or chewable.

The oleic acid plasticizer is substantially tasteless since it is desirable to avoid interfering with the sweet taste generated by the stevia component.

Surprisingly, triglycerides do not plasticize the matrix effectively and so are preferably excluded as plasticizers in the context of the present invention.

The effective amount of oleic acid plasticizer depends on the molecular weight of the ethyl cellulose. Nevertheless, the effective amount is readily and easily ascertained by the person skilled in the art of extrusion through routine work not involving undue burden or experimentation.

As an example though, it is preferred that the weight ratio of oleic acid plasticizer to ethyl cellulose is from 1:48 to 1:2.

For instance, the amount of oleic acid plasticizer is preferably from about 2 to 20% by weight, based on the total weight of the extruded particle.

### Sweetening component

The extruded particle according to the invention comprises a sweetening component selected from the group consisting of stevioside, rebaudioside A, sodium cyclamate, aspartame, sucralose and sodium saccharine, or mixtures thereof. All the aforementioned sweeteners are commercially available. Of these sweetening agents, a stevia component selected from stevioside or rebaudioside A is most preferred

In the context of the present invention, the phrase "Stevia Component" denotes a material that consists of, includes or is derived from the stevia plant. Thus, the Stevia component can be taken to mean the Stevia plant itself, any sweet part thereof, extracts thereof, stevia derivatives, such as steviol glycosides and mixtures thereof.

Stevia or Stevia rebaudiana Bertoni is a sweet-tasting plant. The leaves contain a complex mixture of natural sweet diterpene glycosides. Steviol glycosides, e.g., steviosides and rebaudiosides, are components of Stevia that contribute sweetness. Typically, these compounds are found to include stevioside (4-13% dry weight), steviolbioside (trace), the rebaudiosides, including rebaudioside A (2-4%), rebaudioside B (trace), rebaudioside C (1-2%), rebaudioside D (trace), and rebaudioside E (trace), and dulcoside A (0.4-0.7%). The following nonsweet constituents also have been identified in the leaves of stevia plants: labdane, diterpene, triterpenes, sterols, flavonoids, volatile oil constituents, pigments, gums and inorganic matter.

Suitable stevia derivatives include stevia-based sweetening systems containing a high level of rebaudioside A, a main component contributing to the sweetness of stevia. Thus, it is preferred that the stevia component is a stevia derivative comprising more than 30%, more preferably more than 60%, even more preferably more than 85%, most preferably more than 90%, e.g. more than 95% rebaudioside A by weight based on the total weight of stevia component.

Other suitable stevia derivatives include stevia-based systems comprising a high level of stevioside.

Alternatively, a suitable stevia derivative may comprise both stevioside and rebaudioside A at various ratios.

The sweetening component is preferably present in an amount of from 3 to 40%, more preferably from 5 to 20%, most preferably from 10 to 15% by weight, based on the total weight of the extruded particle.

Surprisingly, it has been found that the beneficial effect of longer lasting flavour or sweetness due to the specific encapsulation system is not achieved when the system comprises a sweetening agent such as neohesperidin dihydrochalcone, or Acesulfame K. In particular, it is found that the release mechanism from a chewing gum base of these sweeteners is not beneficially altered. Thus, the extruded particle of the present invention is preferably substantially free of such sweeteners.

Other ingredients, compounds or compositions may be present in the extruded system. For instance, perfumes, flavours, flavour enhancers, sensate compounds, nutritional ingredients, colours and preservatives are given by way of non-limiting examples.

It is not essential that a flavour is encapsulated to achieve the benefit of longer lasting flavour perception. That is, the flavour can be provided separately from the extruded particle and yet a consumer can still perceive the benefit of prolonged or sustained flavour release.

The extruded particles are formed by a process comprising the steps of extruding a mixture comprising ethyl cellulose, an oleic acid hydrophobic plasticizer and the sweetening component selected from the group consisting of stevioside, rebaudioside A, sodium cyclamate, aspartame, sucralose and sodium saccharine, or mixtures thereof, and granulating the extruded material to form particles.

In a first preferred aspect, the process comprises the steps of:
(i) preparing a substantially homogeneous mixture of ethyl cellulose with the sweetening component, the mixture either being prepared within the extruder or prepared prior to being added into the extruder and then added therein,
(ii) introducing an effective amount of the oleic acid hydrophobic plasticizer into the mixture in the extruder,
(iii) extruding the mixture at a temperature at which the mixture is molten and
(iv) granulating the extruded material to form particles.

In a second preferred aspect, the process comprises the steps of:
(i) preparing a mixture of ethyl cellulose with an effective amount of the oleic acid plasticizer, the mixture either being prepared within the extruder or prepared prior to being added into the extruder and then added therein,
(ii) introducing the sweetening component into the mixture in the extruder,
(iii) extruding the mixture at a temperature at which the mixture is molten and
(iv) granulating the extruded material to form particles.

The advantage of this second process is that it reduces the degree of exposure of the sweetening component, which may be sensitive to heat, to the high temperatures present in the extruder.

Surprisingly, various other methods of producing capsules are not suitable for use with the matrix ingredients used in the present invention. Notably, spray-drying is not compatible since ethyl cellulose is a highly viscous biopolymer which cannot be broken easily into the small droplets that are necessary for the atomization step of spray-drying. Thus, a very large amount of plasticizer would be required and this would create additional difficulties and complications. Furthermore, if it was attempted to use oleic acid as the plasticizer in a spray-drying process, it would not vaporize and a liquid rather than a powder or granule would result.

The extruded particles are preferably incorporated into a foodstuff in order to prolong the perception of flavour or sweetness during consumption or chewing of the foodstuff.

The foodstuff may be any foodstuff where flavour or sweetness intensity is desired to be maintained for a prolonged duration. For instance, the foodstuff may be a chewable product which is kept in the mouth for several minutes. Examples include but are not limited to chewing gums, bubble gum, chewing sticks, chewing pellets and the like. In the context of the present invention, the term "chewing gum" denotes all of the aforementioned chewable products.

Chewing gums, their ingredients and methods of manufacture are well known. For instance, suitable gum bases for use with the extruded particles may be any water-insoluble gum base well known in the art. Illustrative examples of suitable polymers in gum bases include without limitation substances of vegetable origin such as chicle, jelutong, guttapercha and crown gum; synthetic elastomers such as butadiene-styrene copolymer, isobutylene isoprene copolymer, polyethylene, polyisobutylene and polyvinylacetate and the like.

The amount of gum base can vary depending on factors such as the type of base used, consistency desired and other components used to make the final product. Typically, from about 5% to about 45% by weight of gum base based on the total weight of the foodstuff is preferred, more preferably 15% to 30% by weight.

The chewing gum may contain additional conventional additives, including fillers such as calcium carbonate and talc; emulsifiers such as glyceryl monostearate and lecithin; coloring agents such as titanium dioxide and other conventional chewing gum additives known to the person skilled in the art.

The chewing gum composition of the present invention can also include additional other non-encapsulated sweeteners if desired to deliver an instant, but short-lasting, sweetness.

Flavours that can be used in chewing gums include synthetic solid flavouring agents and/or liquids derived from plants, leaves, flowers, fruits and so forth and combinations thereof. Representative flavouring liquids include: spearmint oil, cinnamon oil, oil of wintergreen (methylsalicylate), peppermint oils, natural or synthetic fruit flavours such as citrus oil, including lemon, orange, grape, lime and grapefruit, fruit essences including apple, strawberry, cherry and pineapple can be used.

The amount of flavouring agent employed is normally a matter of preference subject to factors such as flavour type, base type and strength. In general, amounts of 0.5% to about 3% by weight are used in chewing gum compositions with preferred amounts being from about 0.3% to about 1.5%, the most preferred ranges being from 0.7 to about 1.4%.

The encapsulated sweetening component can be added to conventional chewing gum compositions in an amount from about 0.2% to about 8% by weight of the final chewing gum composition, more preferably from about 0.5% to about 5%, most preferably from about 1% to about 3% by weight.

The chewing gum may also comprise a water-soluble bulking agent. For instance, the bulking agent may consist of dextrose, maltose, dextrin, lactose, galactose, polydextrose, sorbitol, mannitol, xylitol or combinations thereof. Such bulking agents are present in an amount ranging from about 30% to about 80% by weight of the entire chewing gum composition.

The chewing gum can be manufactured in any conventional manner, the following merely being an example.

Firstly, the base is heated from about 70° C to about 120° C and placed into a mixer. If coloring is desired, it may be added at this point, followed by the bulking agent, if any, the encapsulated sweetening agent or agents, the gum plasticizing agent and the flavouring agent. When the chewing gum is removed from the mixer, the mixture is rolled or extruded, cut into individual pieces, cooled and then wrapped in a known manner.

### Examples

The invention will now be described with reference to the following examples. It is to be understood that the examples are illustrative of the invention and that the scope of the invention is not limited thereto.

Unless specified to the contrary, samples according to the invention are denoted by a number and comparative examples by a letter. All amounts are % by weight unless otherwise indicated.

### Example 1

### Preparation of Encapsulated Sweeteners

An encapsulated stevia sweetener was prepared comprising the following ingredients in the amounts shown (grams).

**Table 1**

| Ingredient | Amount (grams) |
|---|---|
| Sweetener Component (1) | 105 |
| Ethyl cellulose N7 (2) | 700 |
| Tocopherol,Vitamin E (3) | 2 |
| Oleic acid (4) | 193 |

| | |
|---|---|
| (1) Stevia Rebaudioside A content of 97%, ex Blue California, USA (2) ex Aqualon/ Hercules (3) ex RCA, Germany (4) ex Biodroga, Canada | |

The sweetener component was mixed with the ethyl cellulose powder until homogeneous. The powdery mixture was then introduced at a rate of 820g/hour into an extruder (PRISM 16mm, Thermo Electron, Germany). Simultaneously, the oleic acid was injected at a rate of 178g/hour into the barrel of the extruder through a side port using a gear pump. The screw speed was set at about 350rpm. The temperature of the last two barrels and the die were held at 98°C and 95°C respectively. The die-plate aperture diameter was 1mm.

As the molten extrudate exited the die, it was chopped into discrete particles having an approximate size of 1.5mm using a cutter-knife located at the die-face.

This sweetening system is referred to as "Stevia - example 1".

### Example 2 (comparative example)

### Preparation of comparative encapsulated sweeteners - Acesulfame K (outside the scope of the invention)

The methodology according to example 1 was followed to prepare an encapsulated Acesulfame K sweetening system except that the sweetener was present at 120g and the oleic acid at 178g. This system is referred to as "Acesulfame K- example 2".

### Example 3

### Preparation of encapsulated sweeteners - Aspartame

The methodology according to example 1 was followed to prepare an encapsulated Aspartame sweetening system except that the sweetener was present at 81.7g and the oleic acid at 216.3g. This system is referred to as "Aspartame - example 3".

### Example 4

### Preparation of encapsulated sweeteners - Sucralose

The methodology according to example 1 was followed to prepare an encapsulated Sucralose sweetening system except that the sucralose was present at 99g and the oleic acid at 199g. This system is referred to as "Sucralose - example 4".

### Example 5

### Preparation of encapsulated sweeteners - sodium saccharine

The methodology according to example 1 was followed to prepare an encapsulated sodium saccharine sweetening system except that sodium saccharin was present at 120g and oleic acid at 178g. This system is referred to as "saccharine - example 5".

### Example 6 (comparative example)

### Preparation of comparative encapsulated sweeteners - Neohesperidin dihydrochalcone (outside the scope of the invention)

The methodology according to example 1 was followed to prepare an encapsulated neohesperidin dihydrochalcone sweetening system except that NHDC was present at 50g and the oleic acid at 228g. This system is referred to as "NHDC - example 6".

Unless otherwise stated, the sweeteners used in the examples were purchased from Sigma-Aldrich.

### Example 7

### Preparation of Flavoured Chewing Gums

An unflavoured chewing gum was prepared having the following ingredients in the amounts shown.

**Table 2**

| Ingredient | Amount (wt%) |
|---|---|
| Solsona T Gum Base (1) | 12.46 |
| Vega Gum Base (1) | 12.46 |
| Crystalline sorbitol P60W | 60.8 |
| Maltitol Syrup | 10.3 |
| Glycerin | 3.98 |

| | |
|---|---|
| (1) ex Cafosa | |

To prepare the chewing gum base, a Sigma-blade mixer was pre-heated to 45°C-50°C and half of the polyols were added. The gum base was pre-heated to 60°C-65°C and added to the mixer. Mixing was carried out for approximately 4 minutes. The remaining polyols and humectants were added and mixing was continued for a further 4 minutes.

The unflavoured chewing gum prepared above was then flavoured and sweetened to provide the chewing gum compositions shown in the following two tables:

**Table 3**

| Component | Sample A ** | Sample 1 | Sample B ** | Sample 2 ** | Sample C ** | Sample 3 |
|---|---|---|---|---|---|---|
| Unflavoured Chewing Gum | 96.06 | 96.06 | 95.47 | 95.72 | 94.49 | 94.74 |
| Strawberry Flavour (1) | 1.2 | 1.2 | 1.2 | 1.2 | 1.2 | 1.2 |
| Citric acid | 1.0 | 1.0 | 1.0 | 1.0 | 1.0 | 1.0 |
| ** Stevia (97%) - unencapsulated | 0.2 | - | - | - | - | - |
| Stevia- example 1 | - | 1.90 | - | - | - | - |
| ** Acesulfame K - unencapsulated | - | - | 0.25 | - | - | - |
| ** Acesulfame K - example 2 | - | - | - | 2.08 | - | - |
| ** Aspartame-unencapsulated | - | - | - | - | 0.25 | - |
| Aspartame - example 3 | - | - | | | - | 3.06 |
| ** Empty capsule (2) | 1.90 | - | 2.08 | - | 3.06 | - |

| | | | | | | |
|---|---|---|---|---|---|---|
| ** - denotes comparative examples outside the scope of the invention (1) ex Firmenich, Geneva, Switzerland (reference 744621 02T) (2) Flexarome®, ex Firmenich, Switzerland ref: LAB2594FBS. An extruded particle comprising the same matrix ingredients as examples 1 to 3 but without sweetener encapsulated therein. | | | | | | |

**Table 4**

| Component | Sample D ** | Sample 4 | Sample E ** | Sample 5 | Sample F ** | Sample 6 ** |
|---|---|---|---|---|---|---|
| Unflavoured Chewing Gum | 96.79 | 96.69 | 95.47 | 95.72 | | |
| Strawberry Flavour (1) | 1.2 | 1.2 | 1.2 | 1.2 | 1.2 | 1.2 |
| Citric acid | 1.0 | 1.0 | 1.0 | 1.0 | 1.0 | 1.0 |
| ** Sucralose-unencapsulated | 0.1 | - | - | - | - | - |
| Sucralose - example 4 | - | 1.01 | - | - | - | - |
| ** Saccharin-unencapsulated | - | - | 0.20 | - | - | - |
| Saccharin - example 5 | - | - | - | 1.67 | - | - |
| ** NHDC - unencapsulated | - | - | - | - | 0.04 | - |
| ** NHDC - example 6 | - | - | | | - | 0.80 |
| ** Empty capsule (2) | 1.01 | - | 1.67 | - | 0.80 | - |

| | | | | | | |
|---|---|---|---|---|---|---|
| ** - denotes comparative examples outside the scope of the invention (1) ex Firmenich, Geneva, Switzerland (reference 744621 02T) (2) Flexarome®, ex Firmenich, Switzerland ref: LAB2594FBS. An extruded particle comprising the same matrix ingredients as examples 4 to 6 but without sweetener encapsulated therein. | | | | | | |

To prepare each sample, the unencapsulated sweetener was added to the unflavoured chewing gum preparation and mixed for approximately 2 minutes. The flavour was then added and mixing continued for 2 to 4 minutes. Finally, the encapsulated sweetener (or empty capsule) was added and mixing continued for a further 2 minutes. The sweetened chewing gum was discharged, laminated and cut into sticks or slabs.

Thus, the paired samples (i.e. sample A is paired with sample 1, sample B is paired with sample 2 and so on) had iso-loading of the sweetening agent in the chewing gum.

### Example 8

### Sensory Analysis of Chewing Gums Sweetened with Sweetening Component

18 trained panelists assessed each chewing gum sample for flavour and sweetness intensities at four time intervals: 5, 10, 20 and 30 minutes. Samples were presented blind and following a balanced presentation order. The flavour intensity and sweetness intensity were each evaluated on a scale of 0 to 10 where 0 denotes no flavour or sweetness and 10 denotes very strong flavour or sweetness.

The analysis was performed for each of the paired samples, as indicated below.

### Example 8a

The mean scores for flavour intensity and sweetness intensity for samples A and 1 are given in the following tables.

| Test | Sample A ** | Sample 1 |
|---|---|---|
| Flavour Intensity (time) | Mean | Mean |
| 5 minutes | 4.43 | 4.65 |
| 10 minutes | 2.74 | 3.59 |
| 20 minutes | 1.68 | 2.01 |
| 30 minutes | 1.12 | 1.3 |
| | | |

| Test | Sample A ** | Sample 1 |
|---|---|---|
| Sweetness Intensity (time) | Mean | Mean |
| 5 minutes | 4.15 | 4.75 |
| 10 minutes | 3.54 | 4.43 |
| 20 minutes | 2.62 | 3.5 |
| 30 minutes | 2.14 | 2.67 |

| | | |
|---|---|---|
| ** - denotes comparative examples outside the scope of the invention | | |

### Example 8b

The mean scores for flavour intensity and sweetness intensity for samples B and 2 are given in the following tables.

| Test | Sample B ** | Sample 2 ** |
|---|---|---|
| Flavour Intensity (time) | Mean | Mean |
| 5 minutes | 4.26 | 4.72 |
| 10 minutes | 2.63 | 3.1 |
| 20 minutes | 1.5 | 1.9 |
| 30 minutes | 0.93 | 1.15 |
| | | |

| Test | Sample B ** | Sample 2 ** |
|---|---|---|
| Sweetness Intensity (time) | Mean | Mean |
| 5 minutes | 4.43 | 4.58 |
| 10 minutes | 2.58 | 3.03 |
| 20 minutes | 1.44 | 1.92 |
| 30 minutes | 1.02 | 1.33 |

| | | |
|---|---|---|
| ** - denotes comparative examples outside the scope of the invention | | |

### Example 8c

The mean scores for flavour intensity and sweetness intensity for samples C and 3 are given in the following tables.

| Test | Sample C ** | Sample 3 |
|---|---|---|
| Flavour Intensity (time) | Mean | Mean |
| 5 minutes | 3.92 | 4.40 |
| 10 minutes | 2.58 | 2.97 |
| 20 minutes | 1.71 | 2.01 |
| 30 minutes | 1.14 | 1.45 |
| | | |

| Test | Sample C ** | Sample 3 |
|---|---|---|
| Sweetness Intensity (time) | Mean | Mean |
| 5 minutes | 4.72 | 4.43 |
| 10 minutes | 3.56 | 3.43 |
| 20 minutes | 2.73 | 2.64 |
| 30 minutes | 2.07 | 1.95 |

| | | |
|---|---|---|
| ** - denotes comparative examples outside the scope of the invention | | |

### Example 8d

The mean scores for flavour intensity and sweetness intensity for samples D and 4 are given in the following tables.

| Test | Sample D ** | Sample 4 |
|---|---|---|
| Flavour Intensity (time) | Mean | Mean |
| 5 minutes | 4.67 | 4.66 |
| 10 minutes | 2.88 | 3.36 |
| 20 minutes | 1.68 | 2.09 |
| 30 minutes | 0.98 | 1.57 |
| | | |

| Test | Sample D ** | Sample 4 |
|---|---|---|
| Sweetness Intensity (time) | Mean | Mean |
| 5 minutes | 4.88 | 4.34 |
| 10 minutes | 3.53 | 3.48 |
| 20 minutes | 2.45 | 2.51 |
| 30 minutes | 1.62 | 1.99 |

| | | |
|---|---|---|
| ** - denotes comparative examples outside the scope of the invention | | |

### Example 8e

The mean scores for flavour intensity and sweetness intensity for samples E and 5 are given in the following tables.

| Test | Sample E ** | Sample 5 |
|---|---|---|
| Flavour Intensity (time) | Mean | Mean |
| 5 minutes | 3.55 | 4.83 |
| 10 minutes | 2.2 | 3.25 |
| 20 minutes | 1.35 | 2.14 |
| 30 minutes | 0.84 | 1.46 |
| | | |

| Test | Sample E ** | Sample 5 |
|---|---|---|
| Sweetness Intensity (time) | Mean | Mean |
| 5 minutes | 3.83 | 4.93 |
| 10 minutes | 2.58 | 3.65 |
| 20 minutes | 1.78 | 2.92 |
| 30 minutes | 1.21 | 2.03 |

| | | |
|---|---|---|
| ** - denotes comparative examples outside the scope of the invention | | |

### Example 8f

The mean scores for flavour intensity and sweetness intensity for samples F and 6 are given in the following tables.

| Test | Sample F ** | Sample 6 ** |
|---|---|---|
| Flavour Intensity (time) | Mean | Mean |
| 5 minutes | 3.59 | 3.75 |
| 10 minutes | 2.47 | 2.34 |
| 20 minutes | 1.71 | 1.6 |
| 30 minutes | 1.19 | 1.32 |
| | | |

| Test | Sample F ** | Sample 6 ** |
|---|---|---|
| Sweetness Intensity (time) | Mean | Mean |
| 5 minutes | 4.27 | 3.9 |
| 10 minutes | 2.9 | 2.84 |
| 20 minutes | 1.97 | 2.04 |
| 30 minutes | 1.77 | 1.83 |

| | | |
|---|---|---|
| ** - denotes comparative examples outside the scope of the invention | | |

### Example 9

### LC-MS analysis of chewing gum samples

3 panelists were given various samples of chewing gum prepared above and instructed to chew the gums for 60 minutes. At various intervals, a saliva sample was taken from each panelist, diluted 50 times in water and then analysed by standard LC-MS to confirm the amount of sweetening component remaining in the mouth. The results are as follows:

### Example 9a - Sucralose

| Sample D | Conc. In saliva [µg/g] | | |
|---|---|---|---|
| Time [min] | Subject 1 | Subject 2 | Subject 3 |
| 0.0 | n.d. | - | n.d. |
| 1.0 | 52.8 | - | 133.5 |
| 2.0 | 27.8 | - | 97.7 |
| 3.0 | 24.9 | - | 60.7 |
| 5.0 | 21.1 | - | 31.0 |
| 10.0 | 15.2 | - | 19.6 |
| 15.0 | 11.0 | - | 17.8 |
| 20.0 | 6.3 | - | 13.8 |
| 30.0 | n.d. | - | 7.9 |
| 40.0 | n.d. | - | 5.9 |
| 60.0 | n.d. | - | n.d. |
| | | | |

| Sample 4 | Conc. In saliva [µg/g] | | |
|---|---|---|---|
| Time [min] | Subject 1 | Subject 2 | Subject 3 |
| 0.0 | n.d. | n.d. | n.d. |
| 1.0 | 11.7 | n.d. | 12.1 |
| 2.0 | 23.9 | 5.0 | 22.3 |
| 3.0 | 35.2 | 6.9 | 26.7 |
| 5.0 | 61.6 | 13.1 | 36.0 |
| 10.0 | 59.7 | 19.9 | 29.0 |
| 15.0 | 65.1 | 28.8 | 27.1 |
| 20.0 | 63.0 | 36.1 | 38.6 |
| 30.0 | 38.6 | 23.4 | 28.1 |
| 40.0 | 12.8 | 18.3 | 17.8 |
| 60.0 | n.d. | 9.7 | 11.8 |

The results demonstrate that, in the unencapsulated form, the amount of sucralose diminishes rapidly almost immediately whereas, when encapsulated, it increase during the initial 5 to 10 minutes and then remains remarkably stable for at least until 20 minutes have elapsed.

### Example 9b - Acesulfame K

| Sample B | Conc. In saliva [µg/g] | | |
|---|---|---|---|
| Time [min] | Subject 1 | Subject 2 | Subject 3 |
| 0.0 | n.d. | n.d. | n.d. |
| 1.0 | 295.3 | 212.7 | 228.5 |
| 2.0 | 169.9 | 143.8 | 203.4 |
| 3.0 | 141.6 | 115.2 | 171.0 |
| 5.0 | 79.1 | 61.3 | 99.3 |
| 10.0 | 43.6 | 34.4 | 93.0 |
| 15.0 | 43.3 | 15.6 | 47.6 |
| 20.0 | 28.5 | 6.1 | 29.9 |
| 30.0 | 4.4 | 2.1 | 9.1 |
| 40.0 | 1.2 | n.d. | 3.7 |
| 60.0 | n.d. | n.d. | n.d. |
| | | | |

| Sample 2 | Conc. In saliva [µg/g] | | |
|---|---|---|---|
| Time [min] | Subject 1 | Subject 2 | Subject 3 |
| 0.0 | n.d. | n.d. | n.d. |
| 1.0 | 215.8 | 82.5 | 364.6 |
| 2.0 | 241.4 | 154.7 | 501.6 |
| 3.0 | 287.5 | 165.0 | 449.5 |
| 5.0 | 290.8 | 134.1 | 391.6 |
| 10.0 | 133.0 | 77.6 | 227.1 |
| 15.0 | 95.8 | 72.4 | 224.7 |
| 20.0 | 32.6 | 48.7 | 140.4 |
| 30.0 | 6.5 | 23.8 | 40.7 |
| 40.0 | 3.2 | 5.5 | 12.2 |
| 60.0 | 0.7 | 0.5 | 4.7 |

The results demonstrate that the encapsulation of Acesulfame K has little effect of the release profile of the sweetener compared to the unencapsulated form.

### Example 9c - Aspartame

| Sample C | Conc. In saliva [µg/g] | | |
|---|---|---|---|
| Time [min] | Subject 1 | Subject 2 | Subject 3 |
| 0.0 | n.d. | n.d. | n.d. |
| 1.0 | 22.5 | 2.2 | 77.6 |
| 2.0 | 7.6 | 1.3 | 40.7 |
| 3.0 | 12.2 | 1.9 | 36.8 |
| 5.0 | 14.8 | 4.1 | 71.6 |
| 10.0 | 13.6 | 6.5 | 61.9 |
| 15.0 | 2.0 | 5.0 | 69.1 |
| 20.0 | 0.4 | 5.6 | 21.7 |
| 30.0 | n.d. | 2.1 | 12.0 |
| 40.0 | n.d. | 1.9 | 0.6 |
| 60.0 | n.d. | 1.1 | n.d. |
| | | | |

| Sample 3 | Conc. In saliva [µg/g] | | |
|---|---|---|---|
| Time [min] | Subject 1 | Subject 2 | Subject 3 |
| 0.0 | n.d. | n.d. | n.d. |
| 1.0 | 2.7 | 1.6 | 14.4 |
| 2.0 | 4.0 | 4.2 | 29.0 |
| 3.0 | 5.4 | 3.9 | 30.9 |
| 5.0 | 5.3 | 3.3 | 114.5 |
| 10.0 | 9.3 | 5.2 | 216.2 |
| 15.0 | 93.4 | 4.6 | 132.5 |
| 20.0 | 15.8 | 8.3 | 158.0 |
| 30.0 | 11.9 | 7.7 | 138.5 |
| 40.0 | 4.5 | 2.4 | 51.4 |
| 60.0 | 0.8 | 2.1 | 16.8 |

The results demonstrate that, after 15 minutes, the difference in release profile between unencapsulated and encapsulated form of aspartame becomes significant with the encapsulated form releasing at a surprisingly stable rate for up to 40 minutes.

## Claims

1. An extruded particle for use in a foodstuff, the extruded particle comprising a carrier matrix, the carrier matrix comprising:
ethyl cellulose;
a hydrophobic plasticizer which is oleic acid; and
a sweetening component selected from the group consisting of stevioside, rebaudioside A, sodium cyclamate, aspartame, sucralose and sodium saccharine, or mixtures thereof, dispersed throughout the matrix.

2. The extruded particle according to claim 1, wherein the sweetening component is sucralose.

3. The extruded particle according to any one of the preceding claims wherein the ethyl cellulose is present in an amount of from 95 to 40% by weight, based on the total weight of the extruded particle.

4. The extruded particle according to any one of the preceding claims wherein the weight ratio of the oleic acid hydrophobic plasticizer to ethyl cellulose is from 1:48 to 1:2.

5. The extruded particle according to any one of the preceding claims wherein the sweetening component is present in an amount of from 3 to 40%, by weight, based on the total weight of the extruded particle.

6. A foodstuff comprising an extruded particle according to any one of the preceding claims.

7. A use of an extruded particle according to any one of claims 1 to 5 in a foodstuff to prolong or sustain the perception of a flavour during chewing or consumption of the foodstuff.

8. A method of preparing extruded particles comprising the steps of extruding a mixture comprising ethyl cellulose, a hydrophobic plasticizer which is oleic acid, and a sweetening component selected from the group consisting of stevioside, rebaudioside A, sodium cyclamate, aspartame, sucralose and sodium saccharine, or mixtures thereof, and granulating the extruded material to form particles.

9. The method according to claim 8 comprising the steps of:
(i) preparing a substantially homogeneous mixture of ethyl cellulose with a sweetening component selected from the group consisting of stevioside, rebaudioside A, sodium cyclamate, aspartame, sucralose and sodium saccharine, or mixtures thereof, the mixture either being prepared within the extruder or prepared prior to being added into the extruder and then added therein,
(ii) introducing a plasticizing quantity of a hydrophobic plasticizer which is oleic acid into the mixture in the extruder,
(iii) extruding the mixture at a temperature at which the mixture is molten and
(iv) granulating the extruded material to form particles.

10. The method according to claim 8 comprising the steps of:
(i) preparing a mixture of ethyl cellulose with a plasticizing quantity of a hydrophobic plasticizer which is oleic acid, the mixture either being prepared within the extruder or prepared prior to being added into the extruder and then added therein,
(ii) introducing a sweetening component selected from the group consisting of stevioside, rebaudioside A, sodium cyclamate, aspartame, sucralose and sodium saccharine, or mixtures thereof, into the mixture in the extruder,
(iii) extruding the mixture at a temperature at which the mixture is molten and
(iv) granulating the extruded material to form particles.

11. A method of preparing a foodstuff comprising an extruded particle according to any one of claims 1 to 5, the method comprising the steps of:
(i) preparing extruded particles by the method according to any of claims 8 to 10; and
(ii) incorporating the extruded particles into a foodstuff.

12. The method according to claim 11, wherein the foodstuff is chewing gum.

## Patentansprüche

1. Extrudiertes Partikel zur Verwendung in einem Nahrungsmittel, wobei das extrudierte Partikel eine Trägermatrix umfasst, wobei die Trägermatrix umfasst:
Ethylcellulose;
einen hydrophoben Weichmacher, welcher Ölsäure ist; und
eine Süßungskomponente, ausgewählt aus der Gruppe, bestehend aus Steviosid, Rebaudiosid A, Natriumcyclamat, Aspartam, Sucralose und Natriumsaccharin, oder Gemischen davon, dispergiert überall in der Matrix.

2. Extrudiertes Partikel gemäß Anspruch 1, wobei die Süßungskomponente Sucralose ist.

3. Extrudiertes Partikel gemäß einem der vorhergehenden Ansprüche, wobei die Ethylcellulose in einem Anteil von 95 bis 40 Gew.-%, bezogen auf das Gesamtgewicht des extrudierten Partikels, vorhanden ist.

4. Extrudiertes Partikel gemäß einem der vorhergehenden Ansprüche, wobei das Gewichtsverhältnis des hydrophoben Weichmachers Ölsäure zu Ethylcellulose von 1:48 bis 1:2 beträgt.

5. Extrudiertes Partikel gemäß einem der vorhergehenden Ansprüche, wobei die Süßungskomponente in einem Anteil von 3 bis 40 Gew.-%, bezogen auf das Gesamtgewicht des extrudierten Partikels, vorhanden ist.

6. Nahrungsmittel, umfassend ein extrudiertes Partikel gemäß einem der vorhergehenden Ansprüche.

7. Verwendung eines extrudierten Partikels gemäß einem der Ansprüche 1 bis 5 in einem Nahrungsmittel, um die Wahrnehmung eines Aromas während des Kauens oder des Verzehrs des Nahrungsmittels zu verlängern oder aufrecht zu erhalten.

8. Verfahren zum Herstellen von extrudierten Partikeln, umfassend die Schritte, ein Gemisch, umfassend Ethylcellulose, einen hydrophoben Weichmacher, welcher Ölsäure ist, und eine Süßungskomponente, ausgewählt aus der Gruppe, bestehend aus Steviosid, Rebaudiosid A, Natriumcyclamat, Aspartam, Sucralose und Natriumsaccharin, oder Gemischen davon, zu extrudieren und das extrudierte Material zu granulieren, um Partikel zu erzeugen.

9. Verfahren gemäß Anspruch 8, umfassend die Schritte:
(i) Herstellen eines im Wesentlichen homogenen Gemischs von Ethylcellulose mit einer Süßungskomponente, ausgewählt aus der Gruppe, bestehend aus Steviosid, Rebaudiosid A, Natriumcyclamat, Aspartam, Sucralose und Natriumsaccharin, oder Gemischen davon, wobei das Gemisch entweder innerhalb des Extruders hergestellt wird oder hergestellt wird, bevor es in den Extruder gegeben wird, und dann darin hinzugegeben wird,
(ii) Einführen einer weichmachenden Menge von einem hydrophoben Weichmacher, welcher Ölsäure ist, in das Gemisch in dem Extruder,
(iii) Extrudieren des Gemischs bei einer Temperatur, bei welcher das Gemisch geschmolzen ist, und
(iv) Granulieren des extrudierten Materials, um Partikel zu erzeugen.

10. Verfahren gemäß Anspruch 8, umfassend die Schritte:
(i) Herstellen eines Gemisch von Ethylcellulose mit einer weichmachenden Menge von einem hydrophoben Weichmacher, welcher Ölsäure ist, wobei das Gemisch entweder innerhalb des Extruders hergestellt wird oder hergestellt wird, bevor es in den Extruder gegeben wird, und dann darin hinzugegeben wird,
(ii) Einführen einer Süßungskomponente, ausgewählt aus der Gruppe, bestehend aus Steviosid, Rebaudiosid A, Natriumcyclamat, Aspartam, Sucralose und Natriumsaccharin, oder Gemischen davon, in das Gemisch in dem Extruder,
(iii) Extrudieren des Gemisches bei einer Temperatur, bei welcher das Gemisch geschmolzen ist, und
(iv) Granulieren des extrudierten Materials, um Partikel zu erzeugen.

11. Verfahren zum Herstellen eines Nahrungsmittels, umfassend ein extrudiertes Partikel gemäß einem der Ansprüche 1 bis 5, wobei das Verfahren die Schritte umfasst:
(i) Herstellen extrudierter Partikel durch das Verfahren gemäß einem der Ansprüche 8 bis 10; und
(ii) Einbringen der extrudierten Partikel in ein Nahrungsmittel.

12. Verfahren gemäß Anspruch 11, wobei das Nahrungsmittel Kaugummi ist.

## Revendications

1. Particule extrudée à utiliser dans un produit alimentaire, cette particule extrudée comprenant une matrice support, cette matrice support comprenant:
de l'éthylcellulose;
un ramollissant hydrophobe qui est l'acide oléique; et
un composant édulcorant sélectionné dans le groupe constitué par le stévioside, le rebaudioside A, le cyclamate de sodium, l'aspartame, le sucralose et la saccharine sodique, ou des mélanges de ceux-ci, dispersé dans toute la matrice.

2. Particule extrudée selon la revendication 1, dans laquelle le composant édulcorant est le sucralose.

3. Particule extrudée selon l'une quelconque des revendications précédentes dans laquelle l'éthylcellulose est présente en une quantité de 95 à 40% en poids, sur la base du poids total de la particule extrudée.

4. Particule extrudée selon l'une quelconque des revendications précédentes dans laquelle le rapport pondéral du ramollissant hydrophobe acide oléique à l'éthylcellulose est de 1:48 à 1:2.

5. Particule extrudée selon l'une quelconque des revendications précédentes dans laquelle le composant édulcorant est présent en une quantité de 3 à 40% en poids, sur la base du poids total de la particule extrudée.

6. Produit alimentaire comprenant une particule extrudée selon l'une quelconque des revendications précédentes.

7. Utilisation d'une particule extrudée selon l'une quelconque des revendications 1 à 5 dans un produit alimentaire pour prolonger ou entretenir la perception d'un arôme pendant la mastication ou la consommation du produit alimentaire.

8. Procédé de préparation de particules extrudées comprenant les étapes d'extrusion d'un mélange comprenant de l'éthylcellulose, un ramollissant hydrophobe qui est l'acide oléique, et un composant édulcorant sélectionné dans le groupe constitué par le stévioside, le rebaudioside A, le cyclamate de sodium, l'aspartame, le sucralose et la saccharine sodique, ou des mélanges de ceux-ci, et de granulation du matériau ainsi extrudé pour former des particules.

9. Procédé selon la revendication 8 comprenant les étapes de:
(i) préparation d'un mélange sensiblement homogène d'éthylcellulose avec un composant édulcorant sélectionné dans le groupe constitué par le stévioside, le rebaudioside A, le cyclamate de sodium, l'aspartame, le sucralose et la saccharine sodique, ou des mélanges de ceux-ci, ce mélange étant soit préparé à l'intérieur de l'extrudeuse, soit préparé avant d'être ajouté dans l'extrudeuse puis ajouté dans celle-ci,
(ii) introduction d'une quantité ramollissante d'un ramollissant hydrophobe qui est l'acide oléique dans le mélange présent dans l'extrudeuse,
(iii) extrusion du mélange à une température à laquelle le mélange est fondu et
(iv) granulation du matériau ainsi extrudé pour former des particules.

10. Procédé selon la revendication 8 comprenant les étapes de:
(i) préparation d'un mélange d'éthylcellulose avec une quantité ramollissante d'un ramollissant hydrophobe qui est l'acide oléique, ce mélange étant soit préparé à l'intérieur de l'extrudeuse, soit préparé avant d'être ajouté dans l'extrudeuse puis ajouté dans celle-ci,
(ii) introduction d'un composant édulcorant sélectionné dans le groupe constitué par le stévioside, le rebaudioside A, le cyclamate de sodium, l'aspartame, le sucralose et la saccharine sodique, ou des mélanges de ceux-ci, dans le mélange présent dans l'extrudeuse,
(iii) extrusion du mélange à une température à laquelle le mélange est fondu et
(iv) granulation du matériau ainsi extrudé pour former des particules.

11. Procédé de préparation d'un produit alimentaire comprenant une particule extrudée selon l'une quelconque des revendications 1 à 5, ce procédé comprenant les étapes de:
(i) préparation de particules extrudées au moyen du procédé selon l'une quelconque des revendications 8 à 10; et
(ii) incorporation des particules extrudées dans un produit alimentaire.

12. Procédé selon la revendication 11, dans lequel le produit alimentaire est une gomme à mâcher.
